# EUROPEAN PATENT APPLICATION

(11) **EP 4 692 038 A1**
(43) Date of publication of application: **11.02.2026**
(21) Application number: 24861302.8
(22) Date of filing: 09.07.2024
(51) Int. Cl.: C07C 45/50, C07C 45/82, C07C 47/02

(54) **METHOD FOR PREPARATION OF ALDEHYDE**

(30) Priority: 16.10.2023 KR 20230137652; 04.07.2024 KR 20240087889
(71) Applicant: LG Chem, Ltd., Seoul 07336 (KR)
(72) Inventor: JEONG, Jae Hun, Daejeon 34122 (KR); LEE, Sung Kyu, Daejeon 34122 (KR); KIM, Sung Kyun, Daejeon 34122 (KR); KANG, Min Seok, Daejeon 34122 (KR)
(74) Representative: Goddar, Heinz J.
(86) International application number: PCT/KR2024/009722
(87) International publication number: WO 2025/084544

(57) **Abstract**

Provided is a method for preparing an aldehyde including: reacting reactants in a reactor to obtain a reaction product; supplying the reaction product to a first flash drum, performing gas-liquid separation, and supplying a liquid separated product to a vaporization part including a vaporizer; vaporizing the liquid separated product in the vaporization part to separate a vapor stream and a liquid stream; supplying the vapor stream to a second flash drum to separate an upper discharge stream from the second flash drum and a lower discharge stream from the second flash drum; and supplying the upper discharge stream from the second flash drum to one or more flash drums, and supplying the lower discharge stream from the second flash drum to a distillation tower and performing distillation to obtain the aldehyde from a lower discharge stream from the distillation tower, wherein a ratio of an operating temperature of the vaporizer to a reaction temperature of the reactor is controlled.

## Description

### [Technical Field]

### CROSS-REFERENCE TO RELATED APPLICATIONS

This application claims the benefit of and priority to Korean Patent Application No. 10-2023-0137652 filed on October 16, 2023 and Korean Patent Application No. 10-2024-0087889 filed on July 4, 2024, the entire contents of which are incorporated herein as a part of the specification.

### Technical Field

The present disclosure relates to a method for preparing an aldehyde, and more particularly, to a method for preparing an aldehyde by controlling a ratio of an operating temperature of a vaporizer to a reaction temperature of a reactor.

### [Background Art]

A hydroformylation reaction, which is also known as an oxo reaction, is a reaction in which various olefin-based compounds are reacted with a syngas (carbon monoxide (CO) and hydrogen (H₂)) in the presence of a catalyst to produce a linear (normal) aldehyde and a non-linear (iso) aldehyde having the number of carbon atoms increased by 1.

An aldehyde produced by the oxo reaction may be oxidized or hydrogenated, and converted into acid and alcohol which are aldehyde derivatives, or after a condensation reaction of aldol and the like, may be oxidized or hydrogenated, and converted into various acids and alcohols including a long alkyl group. A hydrogenated alcohol of the aldehyde by the oxo reaction is referred to as an oxo alcohol, and is widely used industrially as various solvents, additives, plasticizer raw materials, synthetic lubricants, and the like.

In the preparation of the aldehyde, high-boiling point by-products such as aldehyde dimers or trimers are produced during the hydroformylation reaction. When these are produced in a large amount and accumulate in the process as impurities, the hydroformylation reaction may be reduced, and process stability is also reduced.

Therefore, a method which may activate the hydroformylation reaction while decreasing accumulation of the high-boiling point by-products is needed.

### [Disclosure]

### [Technical Problem]

In order to solve the problems mentioned in the Background Art, an object of the present disclosure is to provide an effect of decreasing accumulation of high-boiling point by-products and activating the hydroformylation reaction.

However, the object to be solved in the present application is not limited to the object mentioned above, and other objects which are not mentioned may be clearly understood by a person skilled in the art from the following descriptions.

### [Technical Solution]

In one general aspect, provided is a method for preparing an aldehyde that includes: supplying reactants including a syngas and an olefin to a reactor and reacting them in the presence of a catalyst to obtain a reaction product including an aldehyde; supplying the reaction product to a first flash drum, performing gas-liquid separation, and supplying a liquid separated product including an unreacted olefin, the catalyst, and the aldehyde to a vaporization part including a vaporizer; vaporizing the liquid separated product in the vaporization part to separate a vapor stream including the unreacted olefin and the aldehyde, and a liquid stream including the catalyst; supplying the vapor stream to a second flash drum to separate an upper discharge stream from the second flash drum including the unreacted olefin, and a lower discharge stream from the second flash drum including the aldehyde; and supplying the upper discharge stream from the second flash drum to one or more flash drums, and supplying the lower discharge stream from the second flash drum to a distillation tower and performing distillation to obtain the aldehyde from a lower discharge stream from the distillation tower, wherein a ratio of an operating temperature (°C) of the vaporizer to a reaction temperature (°C) of the reactor is 1.1 to 1.9.

### [Advantageous Effects]

According to the method for preparing an aldehyde of the present disclosure, by controlling the ratio of the operating temperature (°C) of the vaporizer to the reaction temperature (°C) of the reactor to 1.1 to 1.9, the amount of the high-boiling point by-products produced during the hydroformylation reaction performed in the reactor and the amount of the high-boiling point by-products further produced in the process may be decreased. Thus, the amount of the high-boiling point by-products included in the liquid stream separated from the vaporization part flash drum circulated to the reactor may be decreased, and a side reaction may be decreased during the hydroformylation reaction. In addition, since the high-boiling point by-products accumulate in a large amount and are circulated in the process, an increase in the operating temperature in the vaporizer and process instability may be suppressed.

Furthermore, since the upper discharge stream from the second flash drum passes through a vacuum pump, internal pressure of the vaporizer is reduced by the vacuum pump, so that a liquid stream and a vapor stream may be separated without an excessive increase in the operating temperature of the vaporizer. Furthermore, the amount of the high-boiling point by-product which is further produced in the vaporizer may be decreased.

An effect which may be obtained in the present application is not limited to the effects mentioned above, and other effects which are not mentioned will be understood clearly by a person with ordinary skill in the art to which the present disclosure pertains from the following description.

### [Description of Drawings]

FIG. 1 is a process flow diagram showing the method for preparing an aldehyde according to an exemplary embodiment of the present disclosure.
FIG. 2 is a process flow diagram showing the method for preparing an aldehyde according to an exemplary embodiment of the present disclosure.

### [Detailed Description]

The terms and words used in the description and claims of the present disclosure are not to be construed limitedly as having general or dictionary meanings but are to be construed as having meanings and concepts meeting the technical ideas of the present disclosure, based on a principle that the inventors are able to appropriately define the concepts of terms in order to describe their own inventions in the best mode.

In the present disclosure, the term "stream" may refer to a fluid flow in a process or may refer to a fluid itself flowing in a pipe. Specifically, the "stream" may refer to both a fluid itself flowing in a pipe connecting each device and a fluid flow. In addition, the fluid may refer to one or more of a solid, a gas, and a liquid.

Meanwhile, in the devices such as a flash drum, a vaporizer, a reactor, and a distillation tower in the present disclosure, unless otherwise particularly stated, a "lower portion" of the device refers to a point at a height of 50% to 100% down from the top of the device, specifically the lowest part (bottom). Likewise, unless otherwise particularly stated, an "upper portion" of the device refers to a point at a height of 0% to 50% down from the top of the device, specifically the top (tower top).

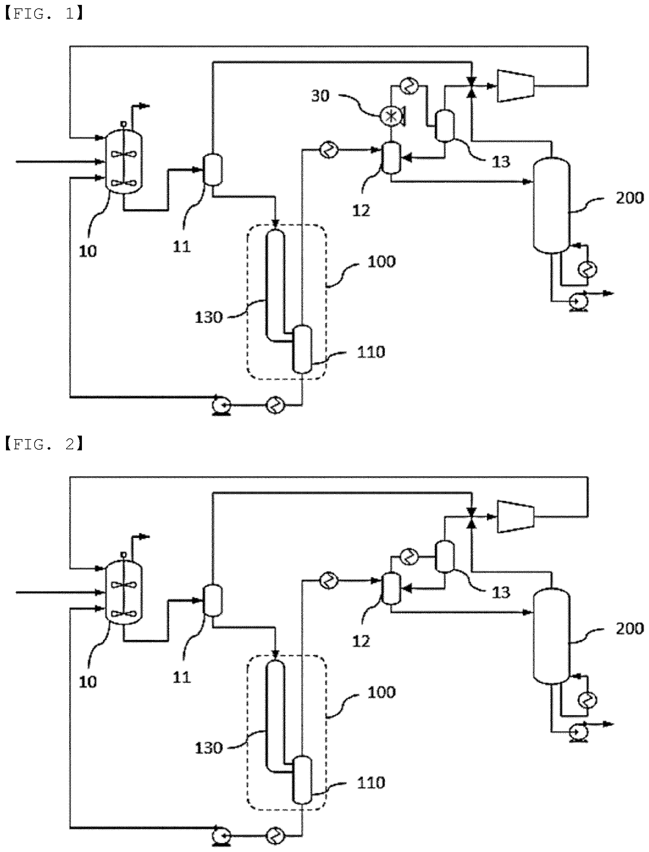

Hereinafter, the present disclosure will be described in more detail with reference to FIG. 1, for better understanding of the present disclosure.

The method for preparing an aldehyde according to an exemplary embodiment of the present disclosure includes: supplying reactants including a syngas and an olefin to a reactor 10 and reacting them in the presence of a catalyst to obtain a reaction product including an aldehyde; supplying the reaction product to a first flash drum 11, performing gas-liquid separation, and supplying a liquid separated product including an unreacted olefin, the catalyst, and the aldehyde to a vaporization part 100 including a vaporizer 130; vaporizing the liquid separated product in the vaporization part 100 to separate a vapor stream including the unreacted olefin and the aldehyde, and a liquid stream including the catalyst; supplying the vapor stream to a second flash drum 12 to separate an upper discharge stream from the second flash drum including the unreacted olefin, and a lower discharge stream from the second flash drum including the aldehyde; and supplying the upper discharge stream from the second flash drum to one or more flash drums, and supplying the lower discharge stream from the second flash drum to a distillation tower 200 and performing distillation to obtain the aldehyde from a lower discharge stream from the distillation tower, wherein a ratio of an operating temperature (°C) of the vaporizer 130 to a reaction temperature (°C) of the reactor 10 is 1.1 to 1.9.

First, the method for preparing an aldehyde according to an exemplary embodiment of the present disclosure may include supplying reactants including a syngas and an olefin to the reactor 10 and reacting them in the presence of a catalyst to obtain a reaction product including an aldehyde.

Specifically, in the reactor 10, a reaction of the syngas and the olefin, that is, a hydroformylation reaction, may be performed. For example, the hydroformylation reaction may be performed by one or more reactors. The hydroformylation reaction may be performed in the presence of a catalyst, and an aldehyde may be produced by the reaction of the syngas and the olefin. That is, a reaction product including the aldehyde produced from the hydroformylation reaction may be obtained.

Meanwhile, unreacted reactants, specifically the syngas, the olefin, and side reactants produced by a side reaction of the hydroformylation reaction, may be included in an upper discharge stream from the reactor and partly vented out of the system. Thus, pressure may be controlled and concentrations of the unreacted substances and side reactants accumulated in gas may be constantly controlled.

The syngas may be a mixed gas of carbon monoxide and hydrogen. A mole ratio between carbon monoxide and hydrogen in the syngas may be 5:95 to 70:30, specifically 40:60 to 60: 40. When the mole ratio in the syngas is within the range, the reaction between the syngas and the olefin in the reactor 10 may be activated.

Meanwhile, the olefin may be an alpha olefin having 3 to 20 carbon atoms, and more specifically, may be an alpha olefin having 3 to 10 or 3 to 8 carbon atoms. For example, the olefin may be one or more selected from ethylene, propylene, butene, 1-hexene, 1-octene, 1-nonene, 1-decene, 1-tetradecene, 1-hexadecene, 1-octadecene, 2-butene, 2-methylpropene, 2-pentene, 2-hexene, 2-octene, and styrene, and preferably, may be propylene. In this case, the catalyst may work excellently, so that production of the aldehyde is further improved. For example, when the olefin is propylene, the aldehyde produced by the hydroformylation reaction may be butyl aldehyde, and more specifically, the butyl aldehyde may include a normal butyl aldehyde and an iso butyl aldehyde.

Meanwhile, the catalyst may include a metal-carbonyl complex catalyst and a ligand. Specifically, the metal-carbonyl complex catalyst may be a catalyst having a transition metal such as cobalt (Co), rhodium (Rh), iridium (Ir), ruthenium (Ru), osmium (Os), platinum (Pt), palladium (Pd), iron (Fe), or nickel (Ni) as a center metal, and preferably, may be a rhodium catalyst. Meanwhile, the ligand may include a trisubstituted phosphine, and specifically, the trisubstituted phosphine may include one or more of triphenylphosphine, tritolylphosphine, triphenylphosphite, and tri-(2-tert-butyl-4-methylphenyl)phosphite. The ligand may be preferably tri-(2-tert-butyl-4-methylphenyl) phosphite. When a rhodium catalyst and tri-(2-tert-butyl-4-methylphenyl)phosphite are used as the catalyst, the hydroformylation reaction performed in the reactor 10 may be more promoted.

Meanwhile, the reaction product may include a catalyst, by-products, an unreacted olefin, and an unreacted syngas, in addition to the aldehyde. Herein, the unreacted olefin and the unreacted syngas may be the olefin and the syngas which have not reacted in the reactor 10.

The method for preparing an aldehyde according to an exemplary embodiment of the present disclosure may include supplying the reaction product to a first flash drum 11, performing gas-liquid separation, and supplying a liquid separated product including an unreacted olefin, the catalyst, and the aldehyde to a vaporization part 100 including a vaporizer 130.

The lower discharge stream from the reactor including the reaction product may be supplied to the first flash drum 11. The lower discharge stream from the reactor may be separated into a gas and a liquid in the first flash drum 11 to obtain a gaseous separated product and a liquid separated product. The gaseous separated product may include the syngas, and the liquid separated product may include the aldehyde. The gaseous separated product may be supplied to one of compressors included in one or more flash drum units described later, and the liquid separated product may be supplied to the vaporization part 100 including the vaporizer 130. Herein, the liquid separated product may further include the catalyst, the by-products, and the unreacted olefin.

The method for preparing an aldehyde according to an exemplary embodiment of the present disclosure may include vaporizing the liquid separated product in the vaporization part 100 to separate a vapor stream including the unreacted olefin and the aldehyde, and a liquid stream including the catalyst.

Specifically, the vaporization part 100 may include the vaporizer 130 and a vaporization part flash drum 110. The vaporization part flash drum 110 may include one or two or more flash drums. Herein, the vaporizer 130 and the vaporization part flash drum 110 included in the vaporization part 100 may be provided, respectively, and connected through a line, or the vaporizer 130 and the vaporization part flash drum 110 may communicate with each other and be connected.

According to an exemplary embodiment of the present disclosure, the vaporization part flash drum 110 may include one flash drum, and the vaporizer 130 may communicate with the vaporization part flash drum 110. Specifically, a lower portion of the vaporizer 130 and an upper portion of the vaporization part flash drum 110 may communicate with each other, that is, may be directly connected without a connection by piping.

More specifically, the liquid separated product is supplied to the vaporizer 130 and a part of the liquid separated product, specifically the unreacted olefin and the aldehyde, may be vaporized. Therefore, the unreacted olefin and the aldehyde which are vaporized in the vaporizer 130, and the catalyst and high-boiling point by-products which are not vaporized, may be separated into a gas and a liquid in the vaporization part flash drum 110. Gaseous components separated in the vaporization part flash drum 110 may be a vapor stream and supplied to the second flash drum 12, and liquid components separated in the vaporization part flash drum 110 may be circulated to the reactor 10 as a liquid stream. Herein, the vapor stream may include the aldehyde and the unreacted olefin, and the liquid stream may include the catalyst and the high-boiling point by-products. Herein, the liquid stream may include some aldehydes which are not separated in the vaporization part flash drum 110.

According to the method for preparing an aldehyde according to an exemplary embodiment of the present disclosure, the ratio of the operating temperature (°C) of the vaporizer 130 to the reaction temperature (°C) of the reactor 10 may be 1.1 to 1.9, specifically 1.3 to 1.7 or 1.3 to 1.5. Herein, the reaction temperature of the reactor 10 may be a temperature at which the hydroformylation reaction is performed in the reactor 10. The operating temperature of the vaporizer 130 may be a lower temperature of the vaporizer 130, and specifically, may be a temperature of a stream which is discharged from the vaporizer 130 and supplied to the vaporization part flash drum 110. For reference, the reaction temperature of the reactor 10 and the operating temperature of the vaporizer 130 for calculating the ratio of the operating temperature of the vaporizer 130 to the reaction temperature of the reactor 10 may be in Celsius.

When the ratio of the operating temperature (°C) of the vaporizer 130 to the reaction temperature (°C) of the reactor 10 is less than 1.1, a temperature at which the liquid separated product is sufficiently vaporized in the vaporization part 110 is not reached, and thus, it may be difficult to obtain an aldehyde which is obtained by vaporizing the liquid separated product. Specifically, when the ratio of the operating temperature (°C) of the vaporizer 130 to the reaction temperature (°C) of the reactor 10 is 1.3 or more, the liquid stream and the vapor stream are effectively separated in the vaporization part 100, thereby increasing the amount of the aldehyde obtained in the distillation tower 200.

Meanwhile, when the ratio of the operating temperature (°C) of the vaporizer 130 to the reaction temperature (°C) of the reactor 10 is more than 1.9, the amount of the high-boiling point by-products produced in the reactor and the vaporization part may be increased. Specifically, when the ratio of the operating temperature (°C) of the vaporizer 130 to the reaction temperature (°C) of the reactor 10 is 1.7 or less, the amount of the high-boiling point by-product included in the liquid stream discharged to the lower portion of the vaporization part flash drum may be decreased, and thus, a conversion rate into an aldehyde in the reactor 10 may be increased.

Meanwhile, for operation of the vaporizer 130, heat may be supplied to the vaporizer 130. Thus, the ratio of the operating temperature (°C) of the vaporizer 130 to the reaction temperature (°C) of the reactor 10 may be controlled to 1.1 to 1.9. Specifically, steam or hot water may be supplied through a line which is separately connected so that heat is supplied to the vaporizer 130, or heat may be supplied to the vaporizer 130 by a fluid at a high temperature in the process. Herein, when the internal pressure of the vaporizer 130 is reduced by a vacuum pump 30 described later, the operating temperature of the vaporizer 130 may not be excessively increased, and thus, the amount of the high-boiling point by-products produced in the vaporization part 100 may be decreased.

Meanwhile, when the operating temperature of the vaporizer 130 is increased, it may be preferred in terms of separation of the liquid stream and the vapor stream in the vaporization part 100, but the amount of the high-boiling point by-products produced in the reactor 10, the vaporizer 130, and the vaporization part flash drum 110 may be increased. In addition, the high-boiling point by-products may be produced in a process of circulating the liquid stream separated in the vaporization part 100 to the reactor 10. Therefore, since the liquid stream having a relatively high amount of the high-boiling point by-products is circulated to the reactor 10, the side reaction of the hydroformylation reaction performed in the reactor 10 may be activated to further increase the amount of the high-boiling point by-products produced. Since the high-boiling point by-product is not separated as a gas phase in the vaporization part 100, the high-boiling point by-products are circulated and accumulated in the process as they are produced, which may reduce operation stability of the process.

Therefore, the operating temperature of the vaporizer 130 needs to be maintained as low as possible, and specifically, the ratio of the operating temperature (°C) of the vaporizer 130 to the reaction temperature (°C) of the reactor 10 needs to be maintained at 1.1 to 1.9. That is to say, in order to reduce the amount of the high-boiling point by-products produced in the reactor 10 and the vaporization part 100 and also improve separation performance in the vaporization part 100, the operating temperature of the vaporizer 130 needs to be lowered to an appropriate level while also setting the internal pressure of the vaporizer 130 low.

According to an exemplary embodiment of the present disclosure, in order to reduce the internal pressure of the vaporizer 130 to a negative pressure, the vacuum pump 30 may be preferably used, and the ratio of the operating temperature (°C) of the vaporizer 130 to the reaction temperature (°C) of the reactor 10 under the reduced pressure conditions by the vacuum pump 30 may be set to 1.7 or less, specifically 1.5 or less.

According to the present disclosure, the internal pressure of the vaporizer 130 may be -0.95 kg/cm².G to 1.0 kg/cm².G, more specifically -0.7 kg/cm².G to 0.8 kg/cm².G, - 0.3 kg/cm².G to 0.2 kg/cm².G, or -0.3 to 0 kg/cm².G, and most preferably -0.3 kg/cm².G to -0.1 kg/cm².G. Herein, the internal pressure of the vaporizer 130 may be gauge pressure.

Meanwhile, the internal pressure of the vaporizer 130 may be controlled to a negative pressure of lower than 0.0 kg/cm².G by the vacuum pump 30 described later. When the internal pressure of the vaporizer 130 is reduced to the negative pressure, the liquid stream and the vapor stream may be effectively separated in the vaporization part 100 without greatly increasing the operating temperature of the vaporizer 130.

The method for preparing an aldehyde according to an exemplary embodiment of the present disclosure may include supplying the vapor stream to a second flash drum 12 to separate an upper discharge stream from the second flash drum including the unreacted olefin, and a lower discharge stream from the second flash drum including the aldehyde.

Specifically, the vapor stream may be supplied to the second flash drum 12, and a condenser may be provided upstream of the second flash drum 12. Therefore, the vapor stream may be condensed through the condenser provided upstream of the second flash drum, and then supplied to the second flash drum 12.

The aldehyde included in the vapor stream may be condensed into a liquid phase by the condensation performed in the condenser. Therefore, the condensed stream discharged from the condenser may include the aldehyde as a condensate and the unreacted olefin as a non-condensate.

Thereafter, the condensed stream may be supplied to the second flash drum 12 and separated into a gas and a liquid. The condensed stream may be separated into an upper discharge stream from the second flash drum including the unreacted olefin, and a lower discharge stream from the second flash drum including the aldehyde in the second flash drum 12.

Subsequently, the upper discharge stream from the second flash drum 12 may be introduced to the vacuum pump 30. That is, when the vacuum pump 30 is provided to be connected to the upper portion of the second flash drum 12, the internal pressure of the second flash drum 12 may be formed to be low by the vacuum pump 30, and efficiency of the gas-liquid separation of the unreacted olefin and the aldehyde performed in the second flash drum 12 may be further improved. Thus, the yield and the production rate of the aldehyde in the subsequent process may be increased.

The method for preparing an aldehyde according to an exemplary embodiment of the present disclosure may include supplying the upper discharge stream from the second flash drum to one or more flash drums, and supplying the lower discharge stream from the second flash drum to a distillation tower 200 and performing distillation to obtain the aldehyde from a lower discharge stream from the distillation tower.

According to the present disclosure, before supplying the upper discharge stream from the second flash drum to the one or more flash drums, the upper discharge stream from the second flash drum may be supplied to the one or more flash drums through the vacuum pump 30.

Specifically, the internal pressure of the vaporizer 130 may be reduced by the vacuum pump 30. More specifically, low pressure may be formed by the vacuum pump 30 through the downstream of the vacuum pump 30, specifically, the second flash drum 12 to the vaporization part 100. The low pressure in the vaporizer 130 means that pressure is controlled to a low state by the vacuum pump 30.

In this case, a liquid stream and a vapor stream may be easily separated in the vaporization part 100 without setting the operating temperature of the vaporizer 130 to be high, as described above. Furthermore, the amount of the high-boiling point by-products produced by the side reaction during the hydroformylation reaction performed in the reactor 10 and the high-boiling point by-products which are further produced in other processes, for example, the vaporization process, performed in the vaporization part 100 may be decreased.

Meanwhile, when the internal pressure of the vaporizer 130 is high, specifically greater than 1.0 kg/cm².G, and more specifically at or above an atmospheric pressure of 0.0 kg/cm².G, the operating temperature of the vaporizer 130 may be increased in order to separate the liquid separated product into a gas and a liquid in the vaporization part. As such, when the operating temperature of the vaporizer 130 is increased, the amount of the high-boiling point by-products produced is increased with the increased temperature, and the increased high-boiling point by-products may be circulated to the reactor in a state of being included in the liquid stream without being separated in the vaporizer 130. The high-boiling point by-products circulated to the reactor 10 may be reacted in the liquid stream to gradually increase the operating temperature of the vaporizer 130, and the high-boiling point by-products may be accumulated in the liquid stream.

Therefore, according to an exemplary embodiment of the present disclosure, the internal pressure of the vaporizer 130 is reduced to a negative pressure through the vacuum pump 30, thereby easily separating the liquid stream and the vapor stream in the vaporization part 100 without excessively increasing the operating temperature of the vaporizer 130.

Meanwhile, the upper discharge stream from the second flash drum may be supplied to one or more flash drums, and specifically, the upper discharge stream from the second flash drum may be supplied to the one or more flash drums through the vacuum pump 30.

The unreacted olefin and the aldehyde included in the second flash drum may be separated into a gas and a liquid by the one or more flash drums, and the unreacted olefin separated in a gas phase may be circulated to the reactor. Therefore, the one or more flash drums may be a flash drum for recovering the unreacted olefin.

The flash drum of the one or more flash drums may be used individually, and may be used by being included in a flash drum unit including the condenser, the flash drum, and the compressor. That is, the flash drum unit may include the flash drum, the condenser provided upstream of the flash drum, and the compressor provided downstream of the flash drum.

Specifically, the flash drum included in the one or more flash drum units separates the condensed stream which has passed through the condenser provided upstream of the flash drum into a gas and a liquid to separate a liquid phase as the lower discharge stream from the flash drum and separate a gas phase as the upper discharge stream from the flash drum. The upper discharge stream from the flash drum may be supplied to a subsequent unit of the flash drum unit to which the flash drum from which the upper discharge stream from the flash drum has been separated belongs, or supplied to the first flash drum 11 via the compressor provided downstream of the flash drum. The compressor provided downstream of the flash drum may serve to compress the gaseous components which are separated into a gas and a liquid in the flash drum. Meanwhile, the lower discharge stream from the flash drum may be supplied to the flash drum included in the front unit of the flash drum unit to which the flash drum from which the lower discharge stream from the flash drum has been separated belongs and one or more units of the units previous to the front units, or supplied to the distillation tower 200 or the second flash drum.

According to an exemplary embodiment of the present disclosure, the one or more flash drums may include a third flash drum 13.

Specifically, a condenser may be provided upstream of the second flash drum 12 and the third flash drum 13, respectively. Herein, the condenser may condense a stream passing through the condenser to condense the aldehyde included in the stream into a liquid phase. Therefore, a condensed stream including the condensed aldehyde through each condenser may be supplied to the second flash drum 12 and the third flash drum 13.

The method for preparing an aldehyde according to an exemplary embodiment of the present disclosure may include supplying the upper discharge stream from the second flash drum to the third flash drum 13 to obtain an upper discharge stream from the third flash drum and a lower discharge stream from the third flash drum, supplying the lower discharge stream from the third flash drum to the second flash drum 12, and circulating the upper discharge stream from the third flash drum to the reactor 10.

Specifically, the upper discharge stream from the second flash drum may be supplied to the third flash drum 13 through the condenser provided upstream of the third flash drum 13. Herein, the upper discharge stream from the third flash drum and the lower discharge stream from the third flash drum may be obtained by gas-liquid separation performed in the third flash drum 13. The lower discharge stream from the third flash drum may be supplied to the second flash drum 12, and the upper discharge stream from the third flash drum may be circulated to the reactor 10.

More specifically, the upper discharge stream from the third flash drum includes the unreacted olefin, and the lower discharge stream from the third flash drum may include the aldehyde. Herein, the upper discharge stream from the third flash drum may include uncondensed components in the condenser provided upstream of the third flash drum 13, and specifically, may include the unreacted olefin. Meanwhile, the lower discharge stream from the third flash drum may include the condensed components in the condenser provided upstream of the third flash drum 13, and specifically, may include the aldehyde.

According to an exemplary embodiment, one or more compressors may be provided downstream of the third flash drum 13. The compressor may serve to lower a pressure difference between the reactor 10 and the upper portion of the third flash drum 13 in order to circulate the upper discharge stream from the third flash drum to the reactor 10.

Meanwhile, the lower discharge stream from the second flash drum may be supplied to the distillation tower 200 and distilled to obtain the lower discharge stream from the distillation tower including the aldehyde, and the upper discharge stream from the distillation tower including the unreacted olefin. The aldehyde may be finally obtained through the lower discharge stream from the distillation tower, and the upper discharge stream from the distillation tower may be supplied to one of the compressors included in the one or more flash drum units, for example, the compressor provided downstream of the third flash drum 13.

Hereinafter, the present disclosure will be described in more detail by the examples. However, the following examples are provided for illustrating the present disclosure, and it is apparent to a person skilled in the art that various modifications and alterations may be made within the scope and the technical idea of the present disclosure, and the scope of the present disclosure is not limited thereto.

### [Examples]

### Example 1

According to the process flow diagram shown in FIG. 1, a preparation process of an aldehyde was simulated, using an Aspen Plus simulator available from Aspen Technology, Inc.

Specifically, reactants including syngas and propylene were supplied to a reactor 10 and reacted in the presence of a catalyst to obtain a reaction product including butyl aldehyde. The reaction product further included an unreacted olefin, the syngas, and high-boiling point by-products.

The reaction product was supplied to a first flash drum 11 and separated into a gas and a liquid to obtain a gaseous separated product including the syngas and a liquid separated product including the unreacted olefin, the catalyst, and the butyl aldehyde. The gaseous separated product was supplied to a compressor provided downstream of the third flash drum 13, and the liquid separated product was supplied to the vaporization part 100.

The liquid separated product was supplied to a vaporizer 130 included in the vaporization part 100 and vaporized, and then was separated into a gas and a liquid in the vaporization part flash drum 110. The upper discharge stream from the vaporization part flash drum including the butyl aldehyde and unreacted propylene, and the lower discharge stream from the vaporization part flash drum including the catalyst and the high-boiling point by-products, were obtained by gas-liquid separation performed in the vaporization part flash drum 110. The operating pressure of the vaporizer 130 was -0.2 kg/cm².G. The operating temperature of the vaporizer 130 was 1.4 to the reaction temperature of the reactor 10. The lower discharge stream from the vaporization part flash drum was circulated to the reactor 10 as a liquid stream, and the upper discharge stream from the vaporization part flash drum was supplied to the condenser provided upstream of the second flash drum 12 as a vapor stream.

The condensed stream obtained from the condenser provided upstream of the second flash drum 12 was separated into a gas and a liquid in the second flash drum 12 to obtain the upper discharge stream from the second flash drum including the unreacted propylene, and the lower discharge stream from the second flash drum including the butyl aldehyde.

The upper discharge stream from the second flash drum was supplied to the condenser provided upstream of the third flash drum 13 through the vacuum pump 30, and the lower discharge stream from the second flash drum was supplied to the distillation tower 200.

The condensed stream obtained from the condenser provided upstream of the third flash drum 13 was separated into a gas and a liquid in the third flash drum 13 to obtain the upper discharge stream from the third flash drum including the unreacted propylene, and the lower discharge stream from the third flash drum including the butyl aldehyde. The upper discharge stream from the third flash drum was circulated to the reactor 10, and the lower discharge stream from the third flash drum was supplied to the second flash drum 12. At this time, the upper discharge stream from the third flash drum was circulated to the reactor 10 through the compressor provided downstream of the third flash drum 13.

The lower discharge stream from the second flash drum described above in the distillation tower 200 was distilled to obtain the upper discharge stream from the distillation tower including the unreacted propylene, and the lower discharge stream from the distillation tower including the butyl aldehyde. The upper discharge stream from the distillation tower was supplied to the compressor provided downstream of the third flash drum 13, and the butyl aldehyde was obtained from the lower discharge stream from the distillation tower.

### Example 2

According to the process flow diagram shown in FIG. 2, a preparation process of an aldehyde was simulated, using an Aspen Plus simulator available from Aspen Technology, Inc.

Specifically, in Example 2, a vacuum pump was not provided unlike Example 1, and at this time, the operating pressure of the vaporizer 130 was 0.0 kg/cm².G, and the operating temperature of the vaporizer 130 was 1.7 to the reaction temperature of the reactor 10.

More specifically, in Example 2, the upper discharge stream from the second flash drum prepared in the same process flow as Example 1 was supplied to the third flash drum 13 through the condenser provided upstream of the third flash drum 13.

The condensed stream obtained through the condenser provided upstream of the third flash drum 13 was separated into a gas and a liquid in the third flash drum 13 to obtain the upper discharge stream from the third flash drum including unreacted propylene, and the lower discharge stream from the third flash drum including butyl aldehyde. The upper discharge stream from the third flash drum was circulated to the reactor 10 through the compressor provided downstream of the third flash drum 13, and the lower discharge stream from the third flash drum was supplied to the distillation tower 200.

As a result, the mass ratio of the high-boiling point by-products included in the lower discharge stream from the vaporization part flash drum of Example 2 was 2.0, based on the mass of the high-boiling point by-products included in the lower discharge stream from the vaporization part flash drum of Example 1.

### Comparative Example 1

An aldehyde was prepared by the same process flow as in Example 1, except that the operating temperature of the vaporizer 130 was controlled to 2.1 to the reaction temperature of the reactor 10.

As a result, the mass ratio of the high-boiling point by-products included in the lower discharge stream from the vaporization part flash drum of Comparative Example 1 was 4.4, based on the mass of the high-boiling point by-products included in the lower discharge stream from the vaporization part flash drum of Example 1.

### Comparative Example 2

The process was performed by the same process flow as in Example 1, except that the operating temperature of the vaporizer 130 was controlled to 0.9 to the reaction temperature of the reactor 10.

As a result, the liquid separated product was not sufficiently vaporized in the vaporizer 130, and it was difficult to separate the vapor stream including the unreacted olefin and the aldehyde and the liquid stream including the catalyst in the vaporization part flash drum 110. Therefore, process operation was difficult.

### [Detailed Description of Main Elements]

10: Reactor
11: First flash drum
12: Second flash drum
13: Third flash drum
30: Vacuum pump
100: Vaporization part
110: Vaporization part flash drum
130: Vaporizer
200: Distillation tower

## Claims

1. A method for preparing an aldehyde, comprising:
supplying a reactant comprising a syngas and an olefin to a reactor and reacting them in the presence of a catalyst to obtain a reaction product comprising an aldehyde;
supplying the reaction product to a first flash drum, performing gas-liquid separation, and supplying a liquid separated product comprising an unreacted olefin, the catalyst, and the aldehyde to a vaporization part comprising a vaporizer;
vaporizing the liquid separated product in the vaporization part to separate a vapor stream comprising the unreacted olefin and the aldehyde, and a liquid stream comprising the catalyst;
supplying the vapor stream to a second flash drum to separate an upper discharge stream from the second flash drum comprising the unreacted olefin, and a lower discharge stream from the second flash drum comprising the aldehyde; and
supplying the upper discharge stream from the second flash drum to one or more flash drums, and supplying the lower discharge stream from the second flash drum to a distillation tower and performing distillation to obtain the aldehyde from a lower discharge stream from the distillation tower,
wherein a ratio of an operating temperature (°C) of the vaporizer to a reaction temperature (°C) of the reactor is 1.1 to 1.9.

2. The method for preparing an aldehyde of claim 1,
wherein before supplying the upper discharge stream from the second flash drum to one or more flash drums, the upper discharge stream from the second flash drum is supplied to the one or more flash drums through a vacuum pump.

3. The method for preparing an aldehyde of claim 2,
wherein an internal pressure of the vaporizer is reduced by the vacuum pump.

4. The method for preparing an aldehyde of claim 1,
wherein the one or more flash drums comprise a third flash drum.

5. The method for preparing an aldehyde of claim 4,
wherein a condenser is provided upstream of the second flash drum and the third flash drum, respectively.

6. The method for preparing an aldehyde of claim 4,
wherein the upper discharge stream from the second flash drum is supplied to the third flash drum to obtain an upper discharge stream from the third flash drum and a lower discharge stream from the third flash drum, and
the lower discharge stream from the third flash drum is supplied to the second flash drum, and the upper discharge stream from the third flash drum is supplied to the reactor.

7. The method for preparing an aldehyde of claim 6,
wherein the upper discharge stream from the third flash drum comprises the unreacted olefin, and
the lower discharge stream from the third flash drum comprises the aldehyde.

8. The method for preparing an aldehyde of claim 1,
wherein internal pressure of the vaporizer is -0.7 to 1.0 kg/cm².G.

9. The method for preparing an aldehyde of claim 1,
wherein the ratio of the operating temperature (°C) of the vaporizer to the reaction temperature (°C) of the reactor is 1.3 to 1.7.

10. The method for preparing an aldehyde of claim 1,
wherein the syngas is a mixed gas comprising carbon monoxide and hydrogen.

11. The method for preparing an aldehyde of claim 1,
wherein the olefin is an alpha olefin having 3 to 20 carbon atoms.
